# EUROPEAN PATENT SPECIFICATION

(11) **EP 4 374 839 B1**
(45) Date of publication and mention of the grant of the patent: **03.12.2025**
(21) Application number: 24160363.8
(22) Date of filing: 29.05.2020
(51) Int. Cl.: A61H 3/00, A61H 1/02

(54) **MEDIAL UNILATERAL EXOSKELETON**
MEDIALES EINSEITIGES EXOSKELETT
EXOSQUELETTE UNILATÉRAL MÉDIAN

(30) Priority: 30.05.2019 CZ 20193340
(43) Date of publication of application: 29.05.2024
(62) Divisional of application: 20731014.5
(73) Proprietor: Mebster S.R.O., 140 00 Praha 4 (CZ)
(72) Inventor: GLOGER, Michal, 70030 Ostrava Hrabuvka (CZ)
(74) Representative: Plasseraud IP

(56) References cited:
- WO-A2-2012/125765
- JP-A- 2003 135 542
- US-A1- 2016 128 890

## Description

### FIELD OF THE DISCLOSURE

The present disclosure generally relates to the field of devices and rehabilitation assistive technologies for persons with severe lower limb immobility, such as paraplegics. The present disclosure is particularly useful for a rehabilitation and a walk enablement of persons with spinal cord injuries.

### BACKGROUND OF THE DISCLOSURE

This section provides background information related to the present disclosure which is not necessarily prior art.

Persons with spinal cord injuries who had a severe or a partial paralysis of the lower limbs need to start with exercise of the paralyzed body segments as soon as possible and verticalize themselves to prevent further health complications due to prolonged sitting such as for instance osteoporosis of the lower limbs, muscle atrophy or bedsores. Such additional health complication causes further discomfort and make the overall treatment very expensive and time consuming.

Passive movement of the lower limbs during rehabilitation can be performed either with an assistance of another person or by a dedicated device. With approximately five hundred thousand spinal cord injuries worldwide each year, resulting in paraplegia in approximately 40% of the injuries, there is a significant need for a universal, inexpensive, easy-to-maintain, and easy-to-use device to allow verticalization of the injured person and his or her gait training with only a limited assistance or even without assistance from third person such as a healthcare professionals.

Currently available rehabilitation assistive technologies include parapodia and conventional lateral exoskeletons consisting of two orthoses, connecting the hips and ankles joints. An example of exoskeletons can be found for instance in US 2016/128890 A1, WO 2012/125765 A2 or JP 2003 135542 A.

US 2016/128890 A1, in accordance with its abstract, states a mobility enhancing device in the form of an exoskeleton that provides mobility assistance or enhancement to a user within the exoskeleton. The exoskeleton may include a torso support and two leg supports coupled to the torso support. Each leg support may include a hip joint, a knee joint, a foot module, and panels connecting the torso support to the hip joints, the hip joints to the knee joints, and the knee joints to the foot modules. Actuators such as motors may be positioned in a discrete location on exoskeleton away from the relatively bulky knee and hip joints to provide for a low profile of the exoskeleton, which may allow the exoskeleton to be worn inconspicuously under a user's clothing.

WO 2012/125765 A2, in accordance with its abstract, states an orthosis that includes a belt assembly, an energy storage apparatus, and an articulable leg frame. The belt assembly is configured to be secured to a user's body. The energy storage apparatus is coupled to the belt assembly and includes a pretension adjustment device and an exotendon. The articulatable leg frame is coupleable to the belt assembly. The energy storage apparatus helps move the user's leg which is coupled to the leg frame.

JP 2003 135542 A, in accordance with its abstract, states problem to be solved: to stabilize and facilitate walking by having a mechanism for shaking out the lower limbs according to the movement of the weight in a knee-ankle- foot orthosis, and setting the foot bottom face to be always parallel with the ground surface even if tilting the lower limbs. Solution: the heel of the left foot, and a first pulley rotated integrally with a right leg, are connected by a first wire. The heel of the right foot, and a second pulley rotated integrally with a left leg, are connected by a second wire. The front foot part of the left foot and the front foot part of the right foot are connected by a third wire.

Typical disadvantages of these assistive technologies are high weight, size and an amount of time it takes don and doff an assistive technology which in most cases requires a need for a third-party assistance. Further drawbacks of the current assistive technologies are time required to adjust an assistive technology to a specific person and importantly an affordability of an assistive technology as in many countries such assistive technologies are not covered by a health insurance and the current assistive technologies are price prohibitive.

Therefore, it would be advantageous to have an assistive technology that takes into account at least some of the issues discussed above as well as possibly other issues.

### SUMMARY OF THE DISCLOSURE

One objective of the present disclosure is to remedy at least part of the above identified drawbacks.

The present disclosure provides a medial unilateral exoskeleton as defined by independent claim 1 and a use of medial unilateral exoskeleton as defined by independent claim 12. Preferred embodiments are defined in the appended dependent claims. Further areas of applicability will become apparent from the description herein. The description and specific examples in the summary are intended for purposes of illustration only and are not intended to limit the scope of the present disclosure.

### BRIEF DESCRIPTION OF THE DRAWINGS

Other features and advantages of the invention appear from the following detailed description of some of its embodiments, given by way of non-limiting example, and with reference to the accompanying drawings, in which:
- **FIG. 1a** is a front view depicting an embodiment of a medial unilateral exoskeleton.
- **FIG. 1b** is a simplified side view depicting the embodiment of the medial unilateral exoskeleton of Figure 1a.
- **FIG. 2a** is a front detail view depicting a detail of an embodiment of the medial hip joint of Figures 1a and 1b.
- **FIG. 2b** is a perspective view of the detail of Figure 2a.
- **FIG. 3** is a side view depicting the arrangement of the ankle joint in the embodiment of Figure 1a.
- **FIG. 4** is a front view depicting the embodiment of the medial unilateral exoskeleton of Figure 1a further equipped with a lumbar orthosis.
- **FIG. 5** is a side view depicting another embodiment of an ankle joint usable in the exoskeleton of Figures 1a or 4, with a moveable element in a first end position.
- **FIG. 6** is a side detail view depicting the embodiment of the ankle joint of Figure 5, with the moveable element in the first end position.
- **FIG. 7** is a perspective view depicting the embodiment of the ankle joint of Figures 5-6, with the moveable element in the first end position.
- **FIG. 8** is a perspective view depicting the embodiment of the ankle joint of Figures 5-7, with the moveable element in a second end position.
- **FIG. 9** is a perspective view depicting another embodiment of a medial unilateral exoskeleton.
- **FIG. 10** is a perspective view depicting the embodiment of the medial unilateral exoskeleton of Figure 9, attached to a person.

### DETAILED DESCRIPTION

The foregoing summary, as well as the following detailed description of certain examples will be better understood when read in conjunction with the appended drawings. As used herein, an element or step recited in the singular and proceeded with the word "a" or "an" should be understood as not excluding plural of the elements or steps, unless such exclusion is explicitly stated. Further, references to "one embodiment" are not intended to be interpreted as excluding the existence of additional embodiments that also incorporate the recited features. Moreover, unless explicitly stated to the contrary, embodiments "comprising" or "having" an element or a plurality of elements having a particular property may include additional elements not having that property.

In the figures, the same references denote identical or similar elements, unless stated otherwise. In the drawings, the size of each element or a specific portion constituting the element is exaggerated, omitted, or schematically shown for convenience and clarity of description. Thus, the size of each component may not entirely reflect the actual size. In the case where it is judged that the detailed description of the related known functions or constructions may unnecessarily obscure the gist of the present disclosure, such explanation will be omitted.

The term medial unilateral exoskeleton relates to a one-sided exoskeleton for placement in the center of a body of a person. Such exoskeleton abuts the lower limbs only from the medial, i.e. the inner side of the limb. That way the medial unilateral exoskeleton ensures a more comfortable use of the exoskeleton for persons that suffer from swelling and / or skin problems where the use of bilateral orthoses that surround the limbs would be more complicated or even excluded.

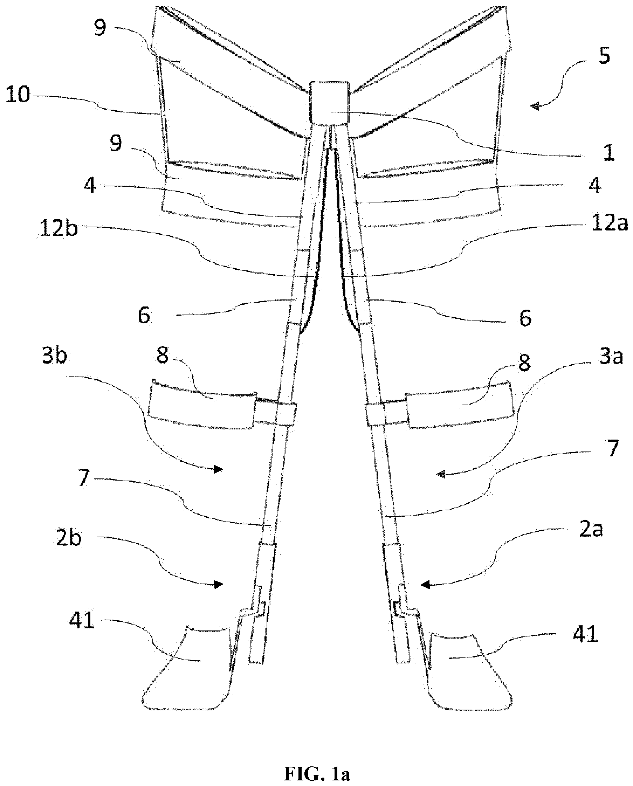

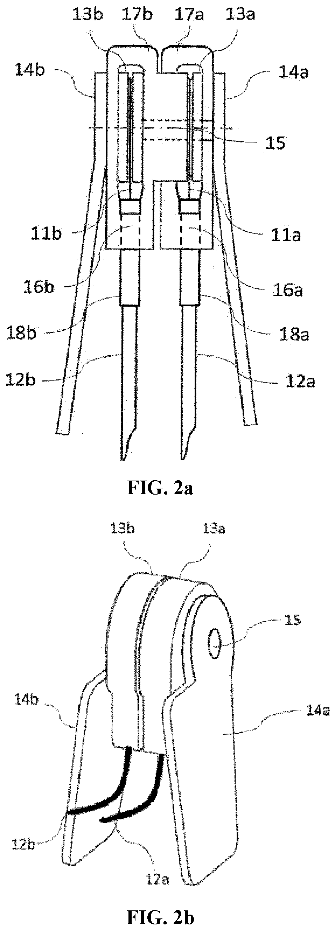

Figure 1a is a front view depicting an embodiment of a medial unilateral exoskeleton. The embodiment of the medial unilateral exoskeleton comprises a medial hip joint 1 comprising the hip plates 14a, 14b and hip pulleys 13a, 13b as shown in Figure 2a and 2b. The embodiment further comprises plurality of actuation cables 12a, 12b and orthoses 3a, 3b. Each actuation cable of the plurality of actuation cables 12a, 12b may be either a pull cable or a push-pull cable and each actuation cable of the plurality of actuation cables 12a, 12b may have a solid or wire rope core. Optionally the actuation cable may be only a wire rope. The solid or wire rope core of the actuation cable may be made from a steel, a stainless steel, a Kevlar, a carbon composite, aramid or any other suitable material. The core may be coated for instance by a PTFE material such as Teflon or the core may be a self-lubricated core to lower friction of the core when please within the cable. Each actuation cable of the plurality of actuation cables 12a, 12b or at least one cable of the plurality of actuation cables may be a Bowden cable. The actuation cables 12a, 12b may be at least partially embedded within the orthoses 3a, 3b.

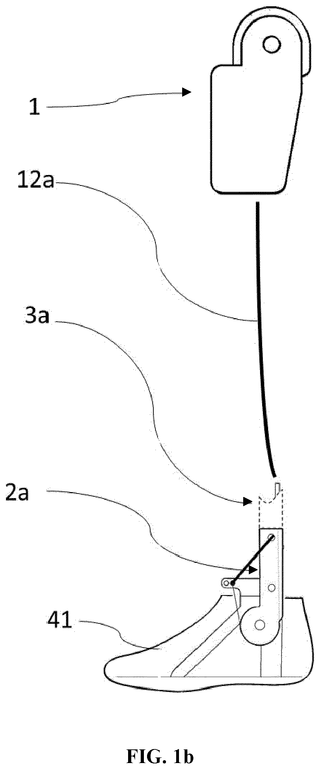

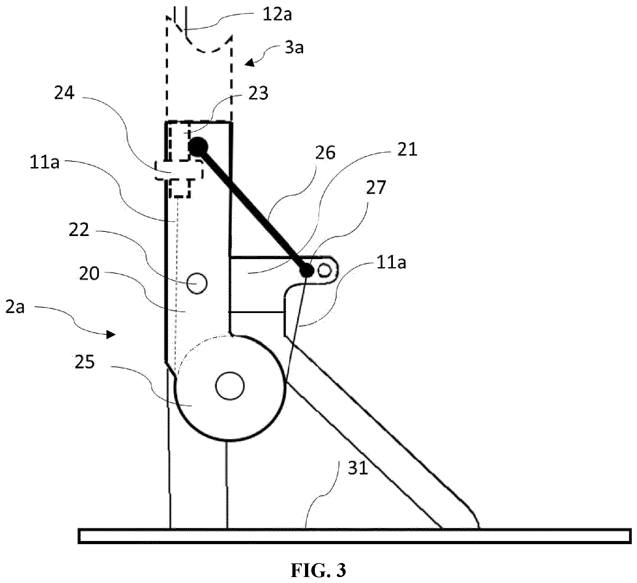

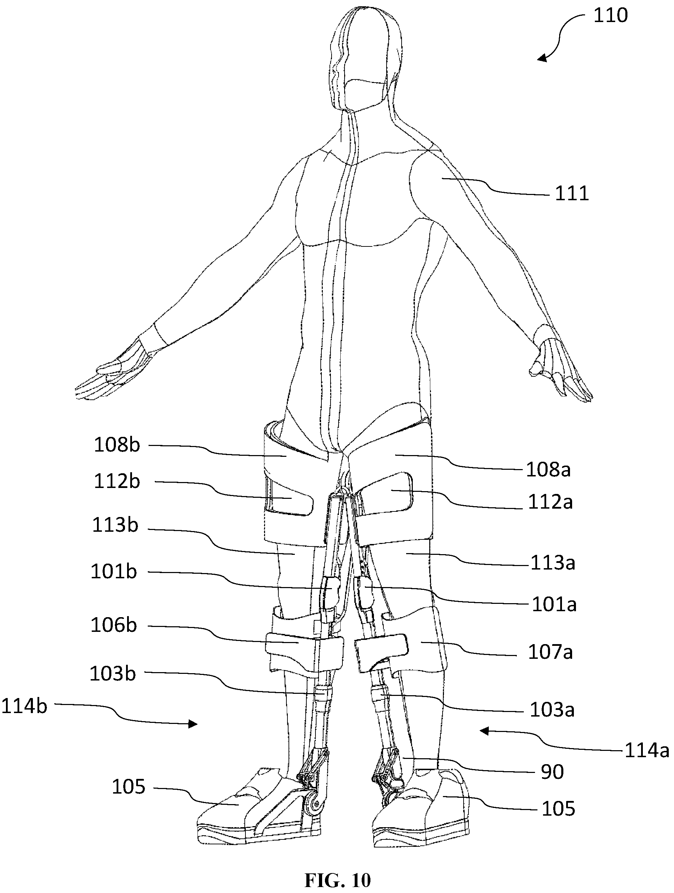

The orthoses may include a right unilateral orthosis 3b, 114b and a left unilateral orthosis 3b, 114a. As shown in Figure 10 the orthoses 114b, 114a are suitable for a right 113b and a left lower limb 113a of a person 111, the orthoses are coupled to the medial hip joint 1 and as depicted in Figure 1a each of the orthoses 3a, 3b comprising an ankle joint 2a, 2b. Each ankle joint 2a, 2b as depicted in Figures 1b and 3a comprises a replaceable stirrup 31 with a shoe 41 or a shoe insert 31, a stirrup coupling 21, an ankle joint body 20 and a guide pulley 25, 68. Each of the ankle joints 2a, 2b as exemplary depicted in Figure 3 may be connected to one of the orthoses 3a, 3b. The stirrup coupling 21 may be rotatably coupled to an ankle pin 22. The ankle pin may be adapted to enable the stirrup coupling to pivot about the axis of the ankle pin 22 in direction d. Such pivot translates into a pivotable movement of the replaceable stirrup 31in relation to the ankle joint body 20. Each of the orthoses may further comprise at least one elastic member 26 having a one end coupled to the ankle joint body 20 and a second end coupled to the stirrup coupling 21. The at least one elastic member 26 may be adapted to act in a second direction opposite to the first direction caused by the movement conveyed by the respective actuation cable 12a, 12b. The elastic member may be formed by one or more elastic elements shaped as a belt, a band, a ring, a string, a rope or a spring or a combination thereof. One benefit of the elastic member is that it enables a more flexible and more natural movement when walking in the medial unilateral exoskeleton as muscles of the person walking in the exoskeleton are more naturally activated whilst the walk stability being maintained. Another benefit of the elastic element is that it can be easily changed or replaced to fit the needs of a person.

Each of the unilateral orthoses further comprises a unilateral medial thigh upright 4, a thigh cuff 5 comprising one or more thigh bands 9, a knee joint 6, an unilateral medial lower leg upright 7 and at least one lower leg cuff 8. The unilateral medial thigh upright 4 and unilateral medial lower leg upright 7 may be either solid or hollow and may have any suitable cross-section, with oval or round cross-sections being preferred, since they do not contain any potentially sharp edges. The unilateral medial thigh upright 4 and unilateral medial lower leg upright 7 may be made of any solid material. For ease of maintenance and handling it advantageous when the material is as light as possible. Suitable materials may be light and / or solid metals or composite materials. The one or more thigh bands 9 and at least one lower leg cuff 8 may be made of any suitable material either that is either flexible or rigid. Suitable materials may be steel, Kevlar or a carbon composite. For fastening to the limb can be used e.g. Velcro or a buckle, or other means that allow tightening bands cuffs to the limb regardless of the size of the circumference of the limb. The one or more thigh cuffs 5 may be replaced by unlocking a locking pin, extending the one or more thigh cuffs and snapping in a different cuffs size. In the same way, the at least one lower leg cuff 8 may be replaced. The knee joint 6 is a self-locking joint. Such self-locking joint may enable the medial unilateral exoskeleton handling as well as it simplifies the attachment of the exoskeleton to a seated person. The self-locking knee joint 6 is adapted to lock the medial thigh upright 4 and the lower leg upright 7 rigidly together. One benefit of the self- locking knee joint is to automatically lock the joint whilst the exoskeleton is attached to a standing person.

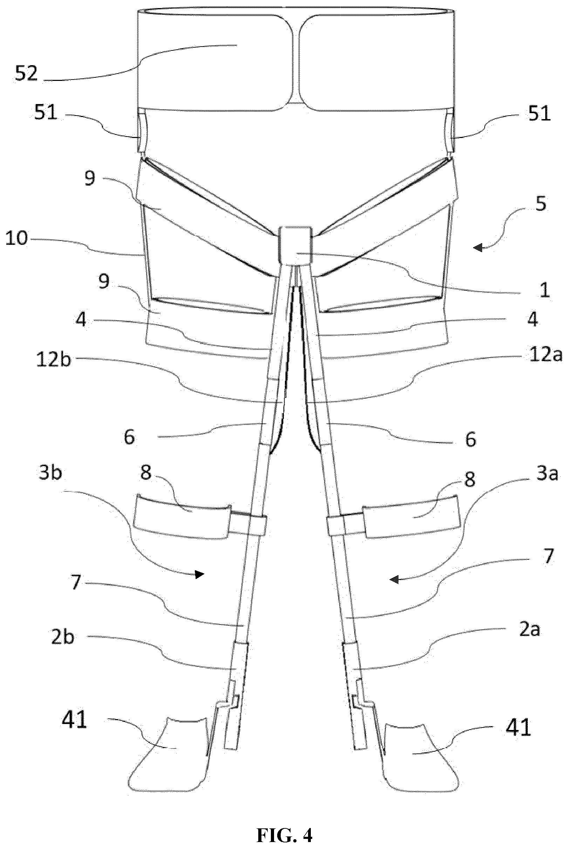

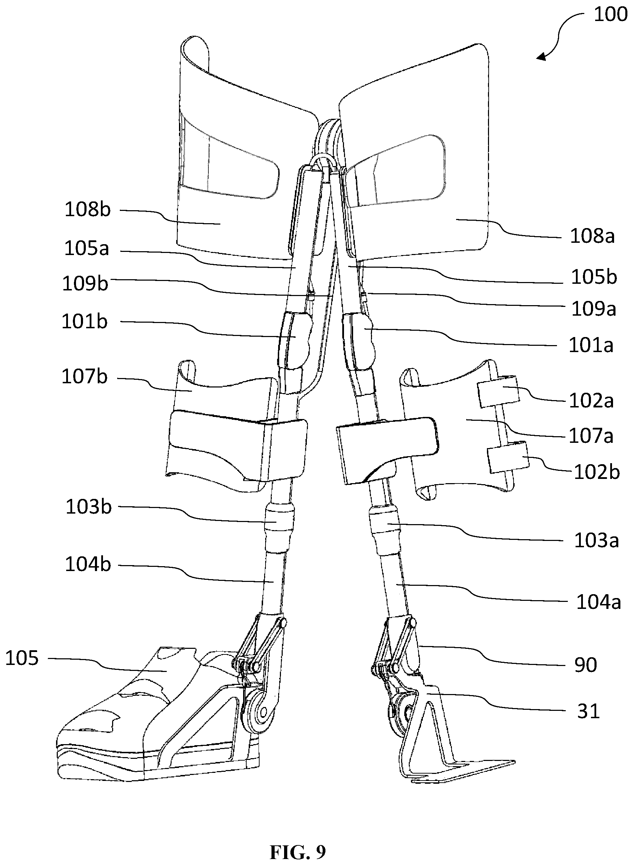

At least one or each of the plurality of actuation cables may be at least partially embedded within the medial lower leg upright 7 and / or the medial thigh upright 4. At least one of the medial thigh upright 4 and / or the medial lower leg upright 7 may have an adjustable length. The adjustable length is controlled by a length adjusting member shown 103a, 103b shown in Figure 9 or 10. Each orthosis may have one or more length adjusting members. As depicted in Figure 4 in one embodiment the medial unilateral exoskeleton may further comprise a lumbar orthosis 52 coupled to the medial hip joint 51 or a lateral hip joint 51. One benefit of such arrangement is a stability improvement of a person having the exoskeleton attached. The lumbar brace connected to the lateral hip joint or medial hip joint may comprise a back support, a bandage and a Velcro fastening and may further include one or more shoulder straps for better spine stabilization.

As shown in Figures 2a, 2b and 3a each of the hip pulleys 13a, 13b may be coupled to a respective ankle joint 2a, 2b by at least one actuation cable 12a, 12b of the plurality of actuation cables so that each actuation cable is on one end attached to one of the hip pulleys 13a, 13b and on a second end attached to the stirrup coupling 21 of the respective ankle joint. Each of the hip pulleys may be coupled to a respective ankle joint by means of one actuation cable of the plurality of actuation cables.

The medial hip joint 1 may connect a medial left and a medial right part of the medial unilateral exoskeleton 5, 100 so that a left ankle joint 2a may be connected to a right side of the medial hip joint and a right ankle joint 2b is connected to a left side of the medial hip joint. The medial hip joint 1 may be in the form of a module where the hip joint with pulleys 13a, 13b is attached to the hip plates 14a, 14b by one or more supporting rollers. This would advantageously enable a rehabilitation facility to easily exchange a hip joint for a more robust option for a person with a higher body weight.

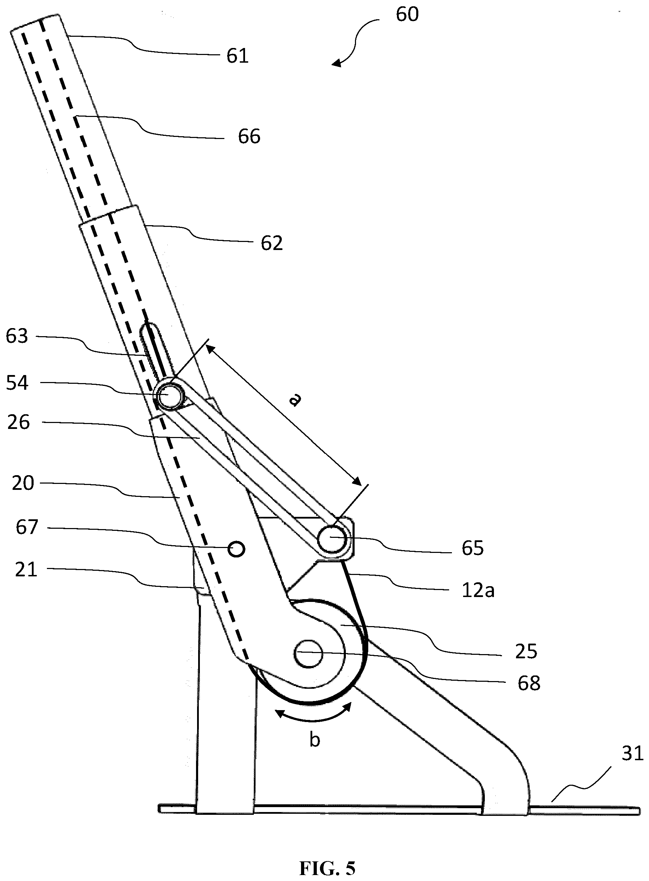

As explained and further shown in Figure 3 the replaceable stirrup may be pivotably connected to the ankle joint body 20 via the stirrup coupling 21. The replaceable stirrup may be pivotably connected to the ankle joint body via the ankle pin 22 and the stirrup coupling may pivot about the axis of the ankle pin 22 in direction d. Such pivot may translate into a pivotable movement of the replaceable stirrup 31in relation to the ankle joint body 20 in direction d. The guide pulley 25, 68 may be coupled to the ankle joint body 20 and configured to support and direct a respective actuation cable 12a, 12b of the plurality of actuation cables to the stirrup coupling 21. The guide pulley 25, 68 may be connected to the ankle join body 20 via a connection member 68 as shown for instance in Figure 5. Each of the actuation cables 12a, 12b may be configured to convey a movement of the hip pulley to the respective ankle joint and cause a pivot of the respective replaceable stirrup in a first direction. Each of the actuation cables 12a,12b may be fixed by means of the guide pulley 25, 68 to the stirrup coupling 21. The guide pulley 25, 68 may be configured to guide the respective actuation cable to the stirrup coupling 21. The guide pulley 25, 68 may rotate in direction b and provide a bearing surface to direct a load conveyed by a respective actuation cable to and from the stirrup coupling 21. The actuation cable may be a Bowden cable having a cable holder attached to a respective ankle joint body 20. Each ankle joint 2a, 2b may be provided with at least one adjustable element 24 formed optionally by an ankle adjusting screw 23 adapted to provide a tension to the respective actuation cable coupled to the ankle joint and impact the angle in the ankle joint.

As depicted in Figure 2a the medial hip joint 1 may be connected to the hip plates 14a, 14b via a shaft 5. Pulleys may be formed by a left pulley 13a and a right pulley 13b and each pulley of the left pulley 13a and right pulley 13b may be connected to one of the two depicted hip plates 14a, 14b so that a left pulley 13b may be connected to a right hip plate 14b and the right pulley 13a may be connected to a left hip plate 14a. Each of the hip plates 14a, 14b may be connected to one of the two orthosis 3a, 3b that may be an unilateral type orthosis. The right hip plate 14b may be connected to a right unilateral orthosis 3b and the left hip plate may be connected to a left unilateral orthosis 3a. The left and right pulleys 13a, 13b may rotate on or with the shaft 5. When the medial unilateral exoskeleton 5, 100, 110 is attached to a person 111 as depicted on Figure 10 the right pulley 13a may be rotate as the left hip 113a of the person 111 rotates and the left pulley 13b may be rotate along with rotation of the right hip 113b of the person 111. Thus, the right pulley 13a may be associated with each rotation of the left hip 113a and the left pulley 13b may be associated with each rotation of the right hip 113b as the person 111 moves the hips. As further depicted in Figure 2a the medial hip joint 1 may be provided with at least one adjustable element formed by a hip adjusting screw 18a, 18b. The at least one adjustable element may be adapted to secure a tension of the actuation cable and impact angle of the respective ankle joint.

The medial hip joint 1 may connect the medial left and right parts of the medial unilateral exoskeleton so that the left ankle joint 2a may be connected to a right side of the hip joint and the right ankle joint 2b may be connected to the left side of the hip joint. The medial hip joint 1 may be delivered in the form of a module where the hip joint with pulleys may be attached to the hip plates by supporting rollers. One benefit would be to enable rehabilitation facilities to carry out a replacement of one hip joint for a more robust one in the case of person with a higher weight.

The left pulley 13a may be connected to the left ankle joint 2a by means of a first actuation cable 12a. When the first actuation cable 12a is actuated it may cause a plantar flexion of a respective left replaceable stirrup 31 and consequently a planar flexion of a shoe 41 that is connected to the left replaceable stirrup 31. Respective elastic member 26 that may connect a respective stirrup coupling 21 and the left ankle joint body 20 may be configured to hold the left replaceable stirrup in a constant dorsal flexion so that the first actuation cable 12a, when actuated, acts against the pull force imposed by the elastic member to the replaceable stirrup 31.

The right pulley 13b may be connected to the right ankle joint 2b by means of a second actuation cable 12b. When the second actuation cable 12b is actuated it may cause a plantar flexion of a respective right replaceable stirrup 31 and consequently a planar flexion of a shoe 41 that is connected to the right replaceable stirrup 31. Respective elastic member 26 that may connect a respective stirrup coupling 21 and the ankle joint body 20 may be configured to hold the right replaceable stirrup in a constant dorsal flexion so that the second actuation cable 12b, when actuated, acts against the pull force imposed by the elastic member to the replaceable stirrup 31.

Due to the contralateral interconnection of the right side of the medial hip joint 1 and a the left ankle joint 2a and the left side of the medial hip joint and the right ankle joint 2b, the ankle torque (in the direction of the dorsal flexion) may drive the contralateral (opposite) limb when attached to the orthosis to swing forward and propone a step forward.

Each ankle joint 2a, 2b may be interconnected with a replaceable shoe 41, 105 or a shoe insert. One of the following combinations may be used; a replaceable stirrup with a fixed shoe, a replaceable stirrup with a replaceable shoe or a replaceable stirrup with a fixed insert suitable for an insertion into any footwear. The replaceable stirrup 31 may be firmly integrated (for instance glued and / or secured by a fastener) with a central portion of the shoe sole. A shoe may also be temporarily attached to the stirrup, for instance via a conventional fastening means such as a tape or a Velcro. Alternatively, a shoe insert may be firmly attached to the replaceable stirrup and the insert may then be placed into any shoe and fixed to a foot of a person simply by tying up shoelaces or otherwise securing shoe. Replaceable inserts or shoes enable the medial unilateral exoskeleton to be adapted to an individual person, with respect to the foot size and /or to the preferred type of a footwear, possibly even to other specific requirements.

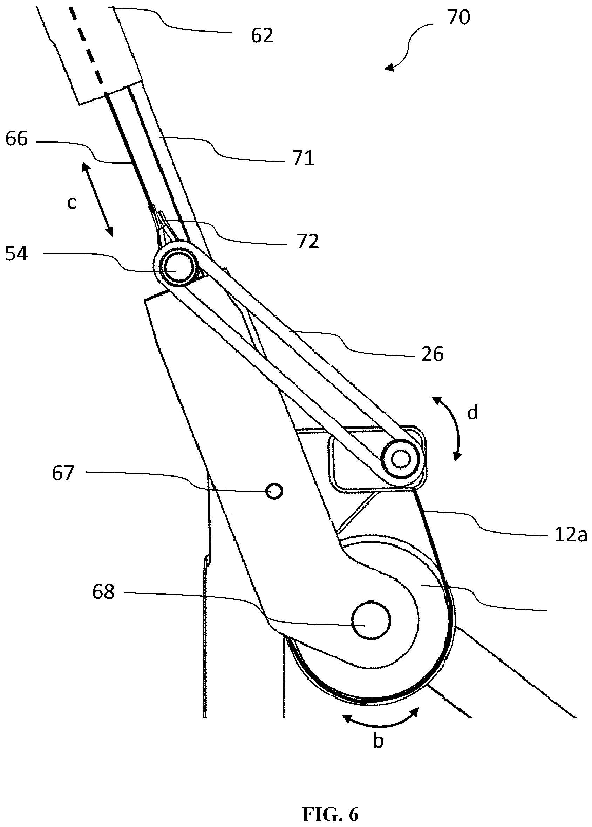

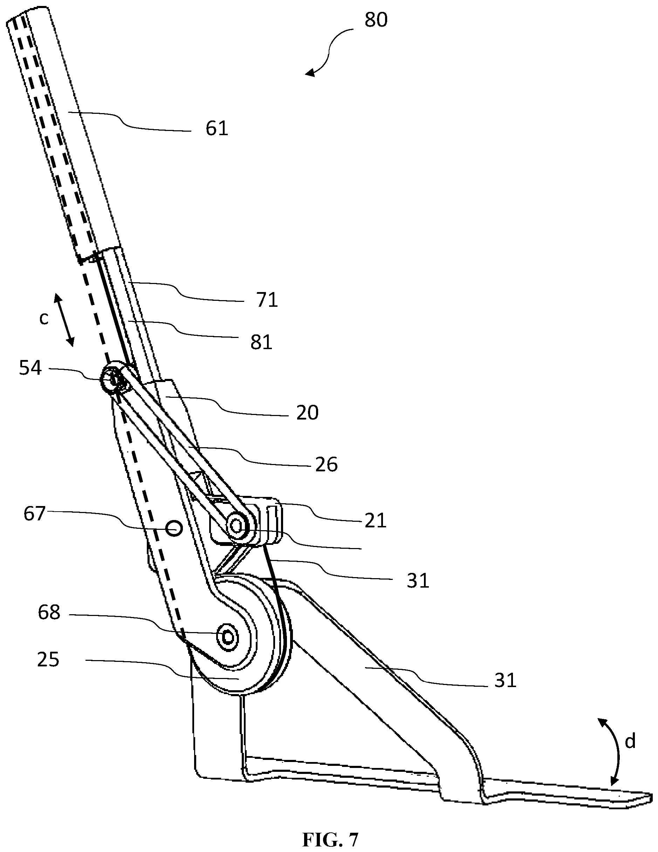

Figures 5 to 8 depict an embodiment of an ankle joint 90 where each of the orthoses further comprises a biasing cable 66 and a moveable member 54. A one end of the elastic member 26 is coupled to the ankle joint body 20 via the moveable member and a one end of the biasing cable 66 is coupled to the moveable member 54. A second end of the biasing cable is coupled to a respective hip pulley 13a, 13b. A second of the elastic member is coupled to the stirrup coupling 21. The second end of the elastic member 26 may by coupled to the stirrup coupling via a holding member 65. The holding member 65 may be rotatably coupled to the stirrup coupling 21. The biasing cable 66 is adapted to position the moveable member so that the distance a between both ends of the elastic member remains constant. One benefit of maintaining same distance between the opposing ends of the elastic member 26 is a constant tension between the ends of the elastic member 26. The constant tension then may be utilized to create a constant pull force imposed by the elastic member 26 to the replaceable stirrup 21.

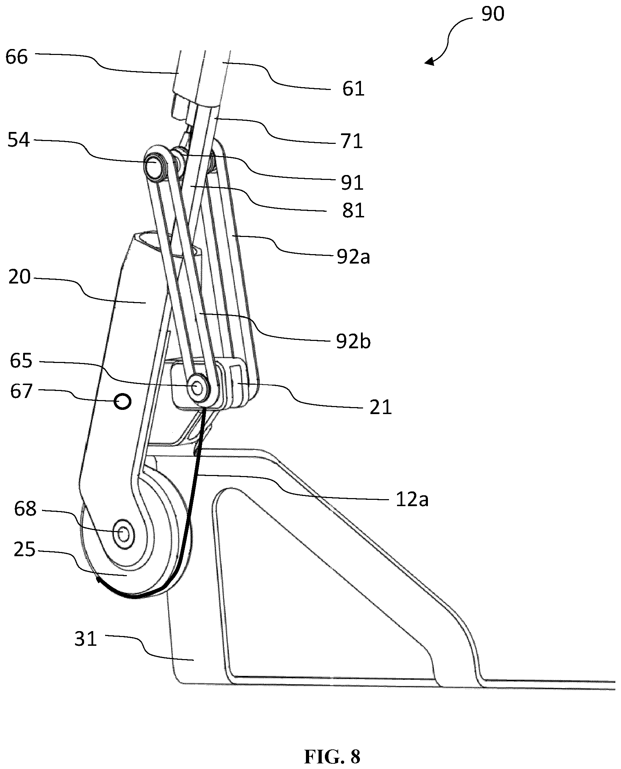

Each of the orthoses may further comprise a linear guide connected to the moveable member. The linear guide may comprise at least one of a rail linear guide, a linear bearing slide, a ball bearing slide, a rack slide, a dovetail slide or a roller slide. Each of the orthoses may further comprise a hollow first part 62 having a slot 63 and a second part 61. The hollow first part 62 may be coupled to the ankle joint body 20 and a portion of the second part 71 may be inserted within the first part 62. A portion of the moveable member may be guided within the slot 63. The portion of the moveable member may engage the portion 71 of the second part 61 within the hollow first part 62. The hollow first part may be a first hollow profile extending in a longitudinal direction and the second part may be a second hollow profile extending in the longitudinal direction. The second part may be slidably engaged into the first hollow profile. The slot 63 may extend in the longitudinal direction. As shown in Figure 8 the moveable member 54 may comprise a roller 91 configured to roll on a guiding profile 81 formed in the portion of the second part 71 within the first hollow part and the guiding profile 81 may extend along the longitudinal direction c. The elastic member 26 may be formed by one or more elastic elements shaped as a belt, a band, a string, a ring, a rope or a spring or a combination thereof. The movable member may extend between two ends perpendicular to the longitudinal direction. The medial unilateral exoskeleton may include two elastic members 92a, 92b which are attached to the ends of the moveable member. One benefit of such arrangement may be to counterbalance roller 91 engaging the guiding profile. Another benefit may be a redundancy of the elastic members as when one of the elastic members is to fail for instance due to is rupture the second elastic member will be able to keep pivotal preload of the stirrup coupling 21 in relations to ankle body.

Figures 9 and 10 depict an exemplary embodiment of a medial unilateral exoskeleton comprising a thigh cuffs 108a, 108b each adapted for an attachment to a thigh 112a, 112b of a person 111. Actuation cables 109a, 109b may be partially embedded into hollow parts of orthoses. Such hollow parts may be medial lower leg upright 104a, 104b and /or medial thigh upright 105a, 105b. The medial lower leg upright 104a, 104b and medial thigh upright 105a, 105b may at least partially to fit to each other. Length adjusting member shown 103a, 103b may be configured to control the partial fit of the medial lower leg upright 104a, 104b and medial thigh upright 105a, 105b and so the length adjusting member is able to control length of the medial thigh upright 105a, 105b and / or the medial lower leg upright 104a, 104b. The medial unilateral exoskeleton may further comprise knee joints 101a, 101b that may be adapted to lock the medial thigh upright 105a, 105b and the lower leg upright 104a, 104b rigidly together. The medial unilateral exoskeleton may further comprise lower leg cuffs 107a and 107b, the lower leg cuffs being adapted to the body proportions of a particular person. Each of the lower leg cuffs may have one or more attachment members 102a, 102b adapted to attached respective lower leg cuff to a leg of a person.

When using the medial unilateral exoskeleton and its variety as described above the orthoses 3a, 114a, 3b, 114b are connected respectively to a right and a left lower limb of a person and the shoe 41, 105 or a shoe insert 41 is connected to a foot of the person.

Such medial unilateral exoskeleton of the present disclosure may be used with crutches, walkers, or parallel walkways. The medial unilateral exoskeleton of the present disclosure is affordable, easy to assemble and maintain, and easy to use. The medial unilateral exoskeleton of the present invention also provides an increased stability and enables safer training of users. The medial unilateral exoskeleton of the present disclosure may be used in a home care because of its easy deployment and relatively simple control which does not require a special assistance from a third party.

### Example 1

In an example a medial unilateral exoskeleton as outlined in previous embodiments may be used as a user specific medial unilateral exoskeleton. Such user specific exoskeleton may comprise a medial hip joint 1 comprising hip plates 14a, 14b, a shaft 15 and pulleys 13a, 13b connected through a unilateral medial thigh upright 4 via the hip plates 14a, 14b to a right and a left lower orthosis. Each of the lower limb orthoses 3a, 3b may comprise, in addition to a metal hollow medial thigh upright 4 having an oval cross section, a thigh cuff 5 comprising two thigh bands 9 connected by a lateral support element 1. Each of the thigh cuffs 5 may be made of a steel thin flat plate provided with a padding and with a Velcro type fastening for an attachment of the thigh cuff to a user's leg. The skeleton may further comprise two self-locking knee joints 6 and two hollow unilateral medial lower leg upright 7 with an oval cross section. Each of the hollow unilateral medial lower leg uprights 7 may be provided with one lower leg cuff 8 in the form of an arc made of a steel thin flat plate provided with a padding and with a Velcro type fastening for an attachment to the user's leg. The length of the each of unilateral medial thigh uprights 4 and the unilateral medial lower leg uprights 7, the circumference of the thigh cuffs 5 and the lower leg cuffs 8 may be adapted to the body proportions of a particular person. Each of the lower limb orthoses 3a, 3b further comprises an ankle joint 2a 2b connected to the pulleys 13a, 13b of the hip joint 1 by means of actuation cables 12a, 12b. The actuation cables may be attached on one end to the respective pulley 13a, 13b of the hip joint 1 and on second end to the stirrup coupling 21 of the respective ankle joints 2a, 2b. The ankle joint body 20 may be further coupled to the stirrup coupling 21 by an elastic element 26. The stirrup coupling 21 of the respective ankle joint 2a, 2b may rotate on a pin 22 of the ankle joint body 2a, 2b. Each of the orthosis 3a, 3b may further comprise a replaceable stirrup 31 that may be connected to an orthopedic shoe 41 of a size corresponding to the leg size of the particular person. Each of the actuation cables 12a, 12b is a Bowden cable that may be attached to a Bowden holder 16 and may be guided partially inside the hollow medial thigh upright 4 and the hollow unilateral medial lower leg upright 7.

Such medial unilateral exoskeleton may be applied by a hip joint 1 between the legs of a seated user. The thigh cuffs 5 may be then attached to the particular user thighs via the thigh bands 9 and the lower leg uprights 7 may be attached to user's calf's via use of one or more lower leg cuffs 8. When the user reaches the vertical position, the thigh plate 4 and the lower leg uprights 7 may be locked at the knee joint 6 so as to hold the user in a vertical position without the possibility of a rotation in the knee joint. Due to the contralateral connection of the left ankle joint with a right hip joint and the right ankle joint with a left hip joint, one foot and associated dorsal flexion contralaterally swings the other limb, reducing effort and allowing the user to move reduced energy intensity and train walking and verticalization. After training in the rehabilitation facility, the user is able to use the medial unilateral exoskeleton using crutches or walkers without the assistance of medical staff.

### Example 2

In another example a medial unilateral exoskeleton as outlined in previous embodiments may be used in a healthcare and / or rehabilitation facility. Such universal medial unilateral exoskeleton may comprise a medial hip joint 1 comprising hip plates 14a, 14b, a shaft 15 and pulleys 13a, 13b that may be covered by pulley support covers 17a, 17b. The medial hip joint may be connected via hip plates 14a, 14b to orthoses 3a, 3b of a right and a left lower limb of a person, specifically through the unilateral medial thigh upright 4. Each of the lower limb orthoses 3a, 3b my include, a medial thigh upright 4 with an oval cross-section, a thigh cuff 5 that may comprise two thigh bands 9 interconnected by a lateral support element 10, the thigh cuff 5 that may be made of thin steel plate. The thigh cuff 5 may be provided with a padding and with a fastening for an attachment of the thigh cuff to a person leg. Each of the orthoses may be further provided with a self-locking knee joint 6, a unilateral medial lower leg upright 7 that may have an oval cross section provided with a one lower leg cuff 8 that may be in the form of a steel thin flat plate provided with a padding and with a Velcro type fastening for an attachment to the person's leg. The length of the medial lower leg upright 7 may be adjustable. The circumference of the thigh cuffs 5 and the lower leg cuff 8 may also be adjustable. Lateral hip joints 51 may be attached to the thigh cuffs 5, to which a lumbar brace 52 may be attached. Each of the lower limb orthoses 3a, 3b may further include an ankle joint 2a, 2b connected to the pulleys 13a, 13b of the hip joint 1 by means of actuation cables 12a 12b. The actuation cables may be attached on one end to the respective pulley 13a, 13b of the hip joint 1 and on second end to the stirrup coupling 21 of the respective ankle joints 2a, 2b.

The ankle joint body 20 may be further coupled to the stirrup coupling 21 by an elastic element 26. The stirrup coupling 21 of the respective ankle joints 2a, 2b rotates on a pin 22 of the ankle joint body 2a, 2b. Each of the orthoses 3a, 3b may further comprise a replaceable stirrup 31 connected to an orthopedic shoe 41. Each of the actuation cables 12a, 12b may be a Bowden cable that may be attached to a Bowden holder 16 and guided partially inside the hollow medial thigh upright 4 and the hollow unilateral medial lower leg uprights 7. The actuation cables 12a, 12b may be replaceable depending on the length required, which is influenced by the adjustment of the length of the medial lower leg upright 7. In the area of the ankle joints 2a, 2b may be located adjustment screws 23 of the actuation cable 12a, 12b that affect the angle in the ankle joint 2a, 2b. A sliding adjustable bar 27 may be formed on the stirrup coupling 21 to influence the transmission ratio between the hip and ankle, further influencing the walking style. In the region of the medial hip joint 2a, 2b, an adjustable element may be provided in the form of a hip adjusting screw 18a, 18b of the actuation cable 12a, 12b. The adjustable element may provide a tension to the actuation cable and affects the angle in the ankle joint 2a, 2b.

The universal medial unilateral exoskeleton may be applied by a hip joint 1 between the legs of a seated user, with the thigh cuffs 5 attached to the persons thighs via the thigh bands 9. The lower leg uprights 7 may be attached to the person calves via one or more lower leg cuffs 8. Next, the lateral hip joint 51 may be attached to the sides of the seated person and the lumbar brace 52 may be attached to the person's body. After the person reaches a vertical position, the medial thigh upright 4 and the lower leg upright 7 may be locked at the knee joint 6 so as to hold the user in a vertical position without the possibility of a rotation within the knee joint. Due to the contralateral connection of the left ankle joint with the right hip joint and the right ankle joint with the left hip joint, one foot and associated dorsal flexion contralaterally swings the other limb, reducing effort and allowing the user to move reduced energy intensity and train walking and verticalization.

While various spatial and directional terms, such as top, bottom, lower, mid, lateral, horizontal, vertical, front and the like may be used to describe embodiments of the present disclosure, it is understood that such terms are merely used with respect to the orientations shown in the drawings. The orientations may be inverted, rotated, or otherwise changed, such that an upper portion is a lower portion, and vice versa, horizontal becomes vertical, and the like.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the disclosure without departing from their scope. While the dimensions and types of materials described herein are intended to define the parameters of the various embodiments of the disclosure, the embodiments are by no means limiting and are exemplary embodiments. Many other embodiments will be apparent to those of skill in the art upon reviewing the above description. The scope of the various embodiments of the disclosure should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

The patentable scope of the various embodiments of the disclosure is defined by the claims, and may include other examples that occur to those skilled in the art. Such other examples are intended to be within the scope of the claims if the examples have structural elements that do not differ from the literal language of the claims.

## Claims

1. A medial unilateral exoskeleton configured to abut lower limbs from the inner side, the exoskeleton comprising:
a medial hip joint (1) comprising hip plates (14a, 14b) and hip pulleys (13a, 13b);
a plurality of actuation cables (11a, 11b);
orthoses (3a, 3b) suitable for a right and a left lower limb of a person, the orthoses are coupled to the medial hip joint and each of the orthoses comprising an ankle joint (2a, 2b) having a replaceable stirrup (31) with a shoe (41) or a shoe insert (41), a stirrup coupling (21), an ankle joint body (20) and a guide pulley (25, 68), wherein each of the hip pulleys (13a, 13b) is coupled to a respective ankle joint (2a, 2b) by at least one actuation cable (11a, 11b) of the plurality of actuation cables so that each actuation cable is on one end attached to one of the hip pulleys (13a, 13b) and on a second end attached to the stirrup coupling of the respective ankle joint, wherein the replaceable stirrup is pivotably connected to the ankle joint body via the stirrup coupling, wherein the guide pulley is coupled to the ankle joint body and configured to support and direct a respective actuation cable of the plurality of actuation cables to the stirrup coupling, wherein each of the actuation cables is configured to convey a movement of the hip pulley to the respective ankle joint via the stirrup coupling and pivot the respective replaceable stirrup in a first direction, wherein each of the orthoses further comprises a unilateral medial thigh upright (4), a thigh cuff (5) comprising one or more thigh bands (9), a self-locking knee joint (6), an unilateral medial lower leg upright (7) and at least one lower leg cuff (8), wherein the self-locking knee joint is adapted to lock the medial thigh upright and the lower leg upright rigidly together when the exoskeleton is attached to a standing person or when the person stands up from a seating position.

2. The medial unilateral exoskeleton of Claim 1, wherein each of the orthoses further comprises at least one elastic member (26) having a one end coupled to the ankle joint body and a second end coupled to the stirrup coupling, wherein the at least one elastic member is adapted to act in a second direction opposite to the first direction caused by the movement conveyed by the respective actuation cable.

3. The medial unilateral exoskeleton according to anyone Claim 1 or 2, wherein each of the actuation cable is either a pull cable or a push-pull cable and each of the actuation cable has a solid or a wire rope core.

4. The medial unilateral exoskeleton according to anyone of Claims 1 to 3, wherein each of the actuation cables or at least one actuation cable of the plurality of actuation cables is a Bowden cable (12a, 12b).

5. The medial unilateral exoskeleton of anyone of Claims 2 to 4, wherein the elastic member is formed by one or more elastic elements shaped as a belt, a band, a string, a rope or a spring or a combination thereof.

6. The medial unilateral exoskeleton of anyone of Claims 1 to 5, wherein each of the hip pulleys is coupled to a respective ankle joint by means of one actuation cable of the plurality of actuation cables.

7. The medial unilateral exoskeleton of Claim 1, wherein each of the actuation cables is at least partially embedded within the medial lower leg upright (7) and / or the medial thigh upright (4).

8. The medial unilateral exoskeleton according to anyone of Claims 1 to 7, wherein the medial thigh upright (4) and / or the medial lower leg upright (7) have an adjustable length.

9. The medial unilateral exoskeleton according to anyone of Claims 1 to 8, wherein the ankle joint is provided with at least one adjustable element formed by an ankle adjusting screw (23) adapted to provide a tension to the actuation cable coupled to the ankle joint and impact the angle in the ankle joint.

10. The medial unilateral exoskeleton according to anyone of Claims 1 to 9, wherein the medial hip joint is provided with at least one adjustable element formed by a hip adjusting screw (18a, 18b), wherein the at least one adjustable element is adapted to secure a tension of the actuation cable and impact angle of the ankle joint.

11. The medial unilateral exoskeleton according to anyone of Claims 1 to 10, further comprising a lumbar orthosis (52) coupled to the medial hip joint (51) or a lateral hip joint.

12. Use of a medial unilateral exoskeleton according to anyone of Claims 1 to 11, wherein the orthoses (3a, 3b) are connected respectively to a right and a left lower limb of a person and the shoe (41) or the shoe insert (41) is connected to a foot of the person.

## Patentansprüche

1. Mediales unilaterales Exoskelett, welches dazu eingerichtet ist, von der inneren Seite an unteren Gliedmaßen anzuliegen, wobei das Exoskelett umfasst:
ein mediales Hüftgelenk (1), welches Hüftplatten (14a, 14b) und Hüftscheiben (13a, 13b) umfasst;
eine Mehrzahl von Betätigungskabeln (11a, 11b);
Orthesen (3a, 3b), welche für eine rechte und eine linke untere Extremität einer Person geeignet sind, wobei die Orthesen mit dem medialen Hüftgelenk gekoppelt sind und jede der Orthesen ein Knöchelgelenk (2a, 2b) umfasst, welches einen austauschbaren Bügel (31) mit einem Schuh (41) oder einer Schuheinlage (41), eine Bügelkopplung (21), einen Knöchelgelenkkörper (20) und eine Führungsscheibe (25, 68) aufweist, wobei jede der Hüftscheiben (13a, 13b) durch wenigstens ein Betätigungskabel (11a, 11b) der Mehrzahl von Betätigungskabeln mit einem jeweiligen Knöchelgelenk (2a, 2b) gekoppelt ist, so dass jedes Betätigungskabel an einem Ende an einer der Hüftscheiben (13a, 13b) angebracht ist und an einem zweiten Ende an der Bügelkopplung des jeweiligen Knöchelgelenks angebracht ist, wobei der austauschbare Bügel über die Bügelkopplung schwenkbar mit dem Knöchelgelenkkörper verbunden ist, wobei die Führungsscheibe mit dem Knöchelgelenkkörper gekoppelt ist und dazu eingerichtet ist, ein jeweiliges Betätigungskabel der Mehrzahl von Betätigungskabeln zu haltern und zu der Bügelkopplung zu lenken, wobei jedes der Betätigungskable dazu eingerichtet ist, eine Bewegung der Hüftscheibe über die Bügelkopplung auf das jeweilige Knöchelgelenk zu übertragen und den jeweiligen austauschbaren Bügel in einer ersten Richtung zu schwenken, wobei jede der Orthesen ferner eine unilaterale mediale Oberschenkelstütze (4), eine Oberschenkelmanschette (5), welche ein oder mehrere Oberschenkelbänder (9) umfasst, ein selbstverriegelndes Kniegelenk (6), eine unilaterale mediale Unterschenkelstütze (7) und wenigstens eine Unterschenkelmanschette (8) umfasst, wobei das selbstverriegelnde Kniegelenk dazu eingerichtet ist, die mediale Oberschenkelstütze und die Unterschenkelstütze starr miteinander zu verriegeln, wenn das Exoskelett an einer stehenden Person angebracht ist oder wenn die Person aus einer sitzenden Position aufsteht.

2. Mediales unilaterales Exoskelett nach Anspruch 1, wobei jede der Orthesen ferner wenigstens ein elastisches Element (26) umfasst, bei welchem ein Ende mit dem Knöchelgelenkkörper gekoppelt ist und ein zweites Ende mit der Bügelkopplung gekoppelt ist, wobei das wenigstens eine elastische Element dazu eingerichtet ist, in eine zweite Richtung zu wirken, welche entgegengesetzt zu der ersten Richtung ist, die durch die Bewegung verursacht wird, welche durch das jeweilige Betätigungskabel übertragen wird.

3. Mediales unilaterales Exoskelett nach Anspruch 1 oder 2, wobei jedes der Betätigungskabel entweder ein Zugkabel oder ein Schubkabel ist und jedes der Betätigungskabel einen festen Kern oder einen Drahtseilkern aufweist.

4. Mediales unilaterales Exoskelett nach einem der Ansprüche 1 bis 3, wobei jedes der Betätigungskabel oder wenigstens ein Betätigungskabel der Mehrzahl von Betätigungskabeln ein Bowdenzug (12a, 12b) ist.

5. Mediales unilaterales Exoskelett nach einem der Ansprüche 2 bis 4, wobei das elastische Element durch ein oder mehrere elastische Elemente gebildet ist, welche als ein Gürtel, ein Band, eine Schnur, ein Seil oder eine Feder oder eine Kombination davon geformt sind.

6. Mediales unilaterales Exoskelett nach einem der Ansprüche 1 bis 5, wobei jede der Hüftscheiben mittels eines Betätigungskabels der Mehrzahl von Betätigungskabeln mit einem jeweiligen Knöchelgelenk gekoppelt ist.

7. Mediales unilaterales Exoskelett nach Anspruch 1, wobei jedes der Betätigungskabel wenigstens teilweise innerhalb der medialen Unterschenkelstütze (7) und/oder der medialen Oberschenkelstütze (4) eingebettet ist.

8. Mediales unilaterales Exoskelett nach einem der Ansprüche 1 bis 7, wobei die mediale Oberschenkelstütze (4) und/oder die mediale Unterschenkelstütze (7) eine einstellbare Länge aufweisen.

9. Mediales unilaterales Exoskelett nach einem der Ansprüche 1 bis 8, wobei das Knöchelgelenk mit wenigstens einem einstellbaren Element bereitgestellt ist, welches durch eine Knöcheleinstellschraube (23) gebildet ist, welche dazu eingerichtet ist, eine Spannung für das Betätigungskabel bereitzustellen, welches mit dem Knöchelgelenk gekoppelt ist, und den Winkel in dem Knöchelgelenk zu beeinflussen.

10. Mediales unilaterales Exoskelett nach einem der Ansprüche 1 bis 9, wobei das mediale Hüftgelenk mit wenigstens einem einstellbaren Element bereitgestellt ist, welches durch eine Hüfteinstellschraube (18a, 18b) gebildet ist, wobei das wenigstens eine einstellbare Element dazu eingerichtet ist, eine Spannung des Betätigungskabels zu sichern und einen Winkel des Knöchelgelenks zu beeinflussen.

11. Mediales unilaterales Exoskelett nach einem der Ansprüche 1 bis 10, ferner umfassend eine Lendenorthose (52), welche mit dem medialen Hüftgelenk (51) oder einem lateralen Hüftgelenk gekoppelt ist.

12. Verwendung eines medialen unilateralen Exoskeletts nach einem der Ansprüche 1 bis 11, wobei die Orthesen (3a, 3b) mit einer rechten bzw. einer linken unteren Extremität einer Person verbunden sind und der Schuh (41) oder die Schuheinlage (41) mit einem Fuß der Person verbunden ist.

## Revendications

1. Exosquelette unilatéral médial configuré pour soutenir des membres inférieurs depuis le côté interne, l'exosquelette comprenant :
une articulation de hanche médiale (1) comprenant des plaques de hanche (14a, 14b) et des poulies de hanche (13a, 13b) ;
une pluralité de câbles d'actionnement (11a, 11b) ;
des orthèses (3a, 3b) appropriées pour un membre inférieur droit et un membre inférieur gauche d'une personne, les orthèses étant accouplées à l'articulation de hanche médiale et chacune des orthèses comprenant une articulation de cheville (2a, 2b) présentant un étrier remplaçable (31) avec une chaussure (41) ou une semelle de chaussure (41), un accouplement à étrier (21), un corps d'articulation de cheville (20) et une poulie de guidage (25, 68), dans lequel chacune des poulies de hanche (13a, 13b) est accouplée à une articulation de cheville (2a, 2b) respective par au moins un câble d'actionnement (11a, 11b) parmi la pluralité de câbles d'actionnement, de sorte que chaque câble d'actionnement soit fixé à une extrémité à l'une des poulies de hanche (13a, 13b) et à une deuxième extrémité à l'accouplement à étrier de l'articulation de cheville respective, dans lequel l'étrier remplaçable est relié de manière pivotante au corps d'articulation de cheville via l'accouplement à étrier, dans lequel la poulie de guidage est accouplée au corps d'articulation de cheville et configurée pour supporter et diriger un câble d'actionnement respectif parmi la pluralité de câbles d'actionnement vers l'accouplement à étrier, dans lequel chacun des câbles d'actionnement est configuré pour transmettre un mouvement de la poulie de hanche à l'articulation de cheville respective via l'accouplement à étrier et faire pivoter l'étrier remplaçable respectif dans une première direction, dans lequel chacune des orthèses comprend en outre un montant de cuisse médial unilatéral (4), un manchon de cuisse (5) comprenant une ou plusieurs bandes de cuisse (9), une articulation de genou à verrouillage automatique (6), un montant de jambe inférieure médial unilatéral (7) et au moins un manchon de jambe inférieure (8), l'articulation de genou à verrouillage automatique étant configurée pour verrouiller ensemble de manière rigide le montant de cuisse médial et le montant de jambe inférieure lorsque l'exosquelette est fixé à une personne debout ou lorsque la personne passe de la position debout à la position assise.

2. Exosquelette unilatéral médial selon la revendication 1, dans lequel chacune des orthèses comprend en outre au moins un organe élastique (26) ayant une extrémité accouplée au corps d'articulation de cheville et une deuxième extrémité accouplée à l'accouplement à étrier, dans lequel l'au moins un organe élastique est adapté pour agir dans une deuxième direction opposée à la première direction sous l'effet du mouvement transmis par le câble d'actionnement respectif.

3. Exosquelette unilatéral médial selon l'une quelconque des revendications 1 ou 2, dans lequel chacun des câbles d'actionnement est soit un câble de traction, soit un câble va-et-vient, et chacun des câbles d'actionnement présente une âme solide ou métallique.

4. Exosquelette unilatéral médial selon l'une quelconque des revendications 1 à 3, dans lequel chacun des câbles d'actionnement ou au moins un câble d'actionnement parmi la pluralité de câbles d'actionnement est un câble bowden (12a, 12b).

5. Exosquelette unilatéral médial selon l'une quelconque des revendications 2 à 4, dans lequel l'organe élastique est formé d'un ou plusieurs éléments élastiques ayant la forme d'une ceinture, d'une bande, d'une ficelle, d'une corde ou d'un ressort, ou d'une combinaison de ceux-ci.

6. Exosquelette unilatéral médial selon l'une quelconque des revendications 1 à 5, dans lequel chacune des poulies de hanche est accouplée à une articulation de cheville respective au moyen d'un câble d'actionnement parmi la pluralité de câbles d'actionnement.

7. Exosquelette unilatéral médial selon la revendication 1, dans lequel chacun des câbles d'actionnement est au moins partiellement intégré dans le montant de jambe inférieure médial (7) et/ou le montant de cuisse médial (4).

8. Exosquelette unilatéral médial selon l'une quelconque des revendications 1 à 7, dans lequel le montant de cuisse médial (4) et/ou le montant de jambe inférieure médial (7) présentent une longueur réglable.

9. Exosquelette unilatéral médial selon l'une quelconque des revendications 1 à 8, dans lequel l'articulation de cheville est dotée d'au moins un élément réglable formé par une vis de réglage de cheville (23) adaptée pour exercer une tension sur le câble d'actionnement accouplé à l'articulation de cheville et influencer l'angle dans l'articulation de cheville.

10. Exosquelette unilatéral médial selon l'une quelconque des revendications 1 à 9, dans lequel l'articulation de hanche médiale est dotée d'au moins un élément réglable formé par une vis de réglage de hanche (18a, 18b), dans lequel l'au moins un élément réglable est adapté pour assurer une tension du câble d'actionnement et influencer l'angle de l'articulation de cheville.

11. Exosquelette unilatéral médial selon l'une quelconque des revendications 1 à 10, comprenant en outre une orthèse lombaire (52) accouplée à l'articulation de hanche médiale (51) ou à une articulation de hanche latérale.

12. Utilisation d'un exosquelette unilatéral médial selon l'une quelconque des revendications 1 à 11, dans laquelle les orthèses (3a, 3b) sont respectivement reliées à un membre inférieur droit et à un membre inférieur gauche d'une personne, et la chaussure (41) ou la semelle de chaussure (41) est reliée à un pied de la personne.
